# EUROPEAN PATENT APPLICATION

(11) **EP 3 991 753 A1**
(43) Date of publication of application: **04.05.2022**
(21) Application number: 19934422.7
(22) Date of filing: 26.06.2019
(51) Int. Cl.: A61K 47/26, A61K 9/10, A61K 47/32, A61K 48/00

(54) **TRANSFECTION METHOD**

(71) Applicant: Takeda Pharmaceutical Company Limited, Osaka-shi, Osaka 541-0045 (JP)
(72) Inventor: MURATA, Naoyuki, Fujisawa-shi, Kanagawa 251-0012 (JP); YANAI, Shigeo, Fujisawa-shi, Kanagawa 251-0012 (JP)
(74) Representative: Jones, Nicholas Andrew
(86) International application number: PCT/JP2019/025506
(87) International publication number: WO 2020/261464

(57) **Abstract**

A novel means for safely and efficiently introducing a target substance such as nucleic acid, protein, or the like into cells (excluding immune cells) is provided by the present invention. Specifically, a system for delivering a target substance into a cell (excluding immune cells), including ultrafine bubble water or ultrafine bubble aqueous solution containing ultrafine bubbles with an average diameter of not more than 200 nm and not containing phospholipid, and an ultrasound generator in combination; a method for increasing the delivery of a nucleic acid, a protein or a low-molecular-weight compound into a cell (excluding immune cells) by using the ultrafine bubble water, etc.; and the like are provided.

## Description

### [Technical Field]

The present invention relates to a system for delivering a target substance into a cell (excluding immune cells), including ultrafine bubble water or an ultrafine bubble aqueous solution containing ultrafine bubbles, and an ultrasonic generator in combination, a method for increasing the delivery of a nucleic acid, a protein or a low-molecular-weight compound into a cell (excluding immune cells) by using the ultrafine bubble water or aqueous solution, and ultrasound, a preparation containing a nucleic acid, a protein or a low-molecular-weight compound, and the ultrafine bubble water or aqueous solution in combination for delivering the nucleic acid, protein or low-molecular-weight compound into a cell (excluding immune cells) by the combined use with ultrasound exposure, and a method for delivering a nucleic acid, a protein or a low-molecular-weight compound into a cell (excluding immune cells) by contacting the cell with the preparation, and treating the cell with ultrasound, and the like.

### (Background of the Invention)

Ultrasound has been mainly used as an ultrasound imaging apparatus in the medical field. Micro-bubble serving as an ultrasound contrast agent is making an epoch-making progress in ultrasound diagnosis. Recently, it has become possible to use ultrasound for purposes other than diagnosis. For example, non-invasive cancer hyperthermic therapy by focusing ultrasonic energy on the affected part and heating only the affected part is clinically applied for hysteromyoma and prostate cancer. In addition, focusing on noninvasiveness and ease of spatial and temporal control, research is also underway to use ultrasound exposure as a tool for drug delivery system (DDS) that delivers genes and drugs to target cells (sonoporation).

Heretofore, as a gene delivery system for skeletal muscle in which bubble liposome and ultrasound technique are fused, a bubble liposome in which perfluoropropane is encapsulated in a polyethylene glycol(PEG)-modified liposome has been reported (non-patent document 1). In addition, a therapeutic drug for Duchenne muscular dystrophy for administering a PEG-modified bubble liposome (bubble lipopolyplex) with a specific morpholino oligomer bound on the surface, encapsulating perfluorohydrocarbon, and having an average particle size of 50 - 500 nm into the muscle tissue or blood vessel, followed by ultrasound exposure to the muscle tissue from outside the body, to achieve highly efficient introduction of the morpholino oligomer into muscle cells has also been reported (patent document 1). It has also been reported that gas-encapsulated micro-bubble and ultrasound can be used for delivering drugs and genes to the brain (non-patent document 2).

It has been reported that bubble liposomes smaller in size than those using perfluorobutane gas or nitrogen gas are obtained using perfluoropropane gas as a filler gas (non-patent document 3). In addition, it has been reported that administration of bubble lipopolyplex, which is a combination of nano-bubbles and plasmid to ddY mice and exposure of ultrasound to the brain tissue produced the introduction of the plasmid into the vascular endothelium or extravascular region, and the introduction site varies depending on the gas encapsulation efficiency (non-patent document 4).

However, the use of bubble liposome is feared to cause problems of antigenicity due to the lipid. In addition, ultrasound exposure at an output intensity of 1.5 to 2.5 W/cm² which poses concerns for safety of ultrasound diagnosis leads to a problem in terms of practicality.

The present inventors have clarified that nano-bubble water containing nano-bubbles not containing phospholipid at not less than 2.0×10⁸ bubbles/mL has a superior antibacterial action (patent document 2). However, there is no report on the introduction of a target substance such as nucleic acid, protein and the like into cells by combining the nano-bubble water and ultrasound.

### [Document List]

### [Patent documents]

patent document 1: WO 2012/153635
patent document 2: WO 2015/182647

### [Non-patent documents]

non-patent document 1: YAKUGAKU ZASSHI 130(11) 1489-1496 (2010)
non-patent document 2: NATURE REVIEWS 12 161-174 (2016)
non-patent document 3: Drug Delivery 24(1) 320-327 (2017)
non-patent document 4: "Evaluation of pharmacokinetics in the brain by ultrasound-responsive nano-bubbles in brain-directed DDS", Yuki Fuchigami et al., Nagasaki University, Abstracts of the 137th Annual Meeting of the Pharmaceutical Society of Japan (March 2017)

### [Summary of Invention]

### [Technical Problem]

An object of the present invention is to provide a novel means for safely and efficiently introducing a target substance such as nucleic acid, protein, or the like into cells (excluding immune cells).

### [Solution to Problem]

To achieve the above-mentioned purpose, the present inventors took note of the fact that the nano-bubble water having a superior antibacterial action, which was developed previously by the present inventors (see the above-mentioned patent document 2; since ISO defines bubbles smaller than 1 µm (1000 nm) as "ultrafine bubbles", they are hereinafter referred to as "ultrafine bubbles" instead of "nano-bubbles" in the present specification) does not contain phospholipid. The ultrafine bubble contained in this ultrafine bubble water has an average diameter of not more than 200 nm which is smaller than the conventional diameter. Conventionally, it has been considered that ultrafine bubbles more severely damage cell membrane than microbubbles when crushed. However, the present inventors dared to combine this ultrafine bubble water with ultrasound exposure and investigated the introduction of nucleic acid and protein into skeletal muscle cells. As a result, it was surprisingly shown that the combination of the ultrafine bubble water and ultrasound can dramatically increases the nucleic acid introduction efficiency as compared with the treatment with any of these alone. The introduction efficiency was superior to that of conventional microbubble liposomes. In addition, it was shown that the sonication can remarkably increase the protein introduction efficiency by the treatment with ultrafine bubble water alone, and that a protein can be efficiently introduced into a cell by the combination of the ultrafine bubble water and ultrasound at a low output intensity of not more than 500 mW/cm².

Based on these findings, the present inventors have further studied and completed the present invention.

That is, the present invention relates to
[1] a system for delivering a target substance into a cell (excluding immune cells), comprising ultrafine bubble water or ultrafine bubble aqueous solution comprising ultrafine bubbles with an average diameter of not more than 200 nm and not containing phospholipid, and an ultrasound generator in combination;
[2] the system of [1], wherein the ultrafine bubble aqueous solution comprises one or more components selected from one or more kinds of surfactants selected from an anionic surfactant, a non-ionic surfactant, a cationic surfactant and an amphoteric surfactant, a hydrophilic resin, and a buffer;
[3] the system of [1] or [2], wherein the ultrafine bubble aqueous solution consists of a non-ionic surfactant and/or a hydrophilic resin;
[4] the system of any of [1] to [3], wherein the ultrafine bubble is constituted of perfluorohydrocarbon or air;
[5] the system of any of [1] to [4], wherein the ultrafine bubble has an average diameter of 50 nm - 200 nm;
   [5-1] the system of any of [1] to [4], wherein the ultrafine bubble has an average diameter of 75 nm - 165 nm;
[6] the system of any of [1] to [5], wherein the ultrafine bubble has a d90/d10 ratio of not more than 5;
   [6-1] the system of any of [1] to [5-1], wherein the ultrafine bubble has a d90/d10 ratio of not more than 4;
[7] the system of any of [1] to [6-1], wherein the ultrafine bubble in the ultrafine bubble water or ultrafine bubble aqueous solution has a density of not less than 1.0×10⁸ bubbles/mL;
   [7-1] the system of any of [1] to [6-1], wherein the ultrafine bubble in the ultrafine bubble water or ultrafine bubble aqueous solution has a density of 1.0×10⁸ - 2.0×10⁹ bubbles/mL;
[8] the system of any of [1] to [7-1], wherein ultrasound output intensity in the ultrasound generator is not more than 720 mW/cm²;
[9] the system of any of [1] to [8], wherein in the ultrasound generator, ultrasound output intensity is 50 - 500 mW/cm² and ultrasound frequency is 0.5 - 10 MHz;
[10] the system of any of [1] to [9], wherein the target substance is a nucleic acid, a protein or a low-molecular-weight compound;
   [10A] the system of any of [1] to [9], wherein the target substance nucleic acid has a molecular weight of 10 mer - 30 mer;
   [10B] the system of any of [1] to [9], wherein the target substance protein has a molecular weight of 25 kDa - 900 kDa;
   [10C] the system of any of [1] to [9], wherein the target substance has a molecular weight of not less than 25 kDa, and the ultrafine bubble is positively charged;
   [10D] the system of any of [1] to [9], wherein the target substance has a molecular weight of less than 25 kDa;
[11] the system of any of [1] to [10D], wherein the cell is a skeletal muscle cell or a nerve cell;
[12] a method for increasing the delivery of a nucleic acid, a protein or a low-molecular-weight compound into a cell (excluding immune cells) by using ultrafine bubble water or ultrafine bubble aqueous solution comprising ultrafine bubble with an average diameter of not more than 200 nm and not containing phospholipid, and ultrasound;
   [12-1] the method of [12], wherein the ultrafine bubble aqueous solution comprises one or more components selected from one or more kinds of surfactants selected from an anionic surfactant, a non-ionic surfactant, a cationic surfactant and an amphoteric surfactant, a hydrophilic resin, and a buffer;
   [12-2] the method of [12] or [12-1], wherein the ultrafine bubble aqueous solution consists of a non-ionic surfactant and/or a hydrophilic resin;
   [12-3] the method of any of [12] to [12-2], wherein the ultrafine bubble is constituted of perfluorohydrocarbon or air;
   [12-4] the method of any of [12] to [12-3], wherein the ultrafine bubble has an average diameter of 50 nm - 200 nm;
   [12-5] the method of any of [12] to [12-3], wherein the ultrafine bubble has an average diameter of 75 nm - 165 nm;
   [12-6] the method of any of [12] to [12-5], wherein the ultrafine bubble has a d90/d10 ratio of not more than 5;
   [12-7] the method of any of [12] to [12-5], wherein the ultrafine bubble has a d90/d10 ratio of not more than 4;
   [12-8] the method of any of [12] to [12-7], wherein the ultrafine bubble in the ultrafine bubble water or ultrafine bubble aqueous solution has a density of not less than 1.0×10⁸ bubbles/mL;
   [12-9] the method of any of [12] to [12-7], wherein the ultrafine bubble in the ultrafine bubble water or ultrafine bubble aqueous solution has a density of 1.0×10⁸ - 2.0×10 bubbles/mL;
   [12-10] the method of any of [12] to [12-9], wherein ultrasound output intensity in the ultrasound generator is not more than 720 mW/cm²;
   [12-11] the method of any of [12] to [12-9], wherein in the ultrasound generator ultrasound output intensity is 50 - 500 mW/cm² and ultrasound frequency is 0.5 - 10 MHz;
   [12-12] the method of any of [12] to [12-11], wherein the target substance is a nucleic acid, a protein or a low-molecular-weight compound;
   [12-12A] the method of any of [12] to [12-11], wherein the target substance nucleic acid has a molecular weight of 10 mer - 30 mer;
   [12-12B] the method of any of [12] to [12-11], wherein the target substance nucleic acid has a molecular weight of 25 kDa - 900 kDa;
   [12-12C] the method of any of [1] to [9], wherein the target substance nucleic acid has a molecular weight of not less than 25 kDa, and the ultrafine bubble is positively charged;
   [12-12D] the method of any of [1] to [9], wherein the target substance has a molecular weight of less than 25 kDa;
   [12-13] the method of any of [12] to [12-12D], wherein the cell is a skeletal muscle cell or a nerve cell;
[13] a preparation comprising a combination of a nucleic acid, a protein or a low-molecular-weight compound, and ultrafine bubble water or ultrafine bubble aqueous solution comprising ultrafine bubble with an average diameter of not more than 200 nm and not containing phospholipid, for delivering the nucleic acid, protein or low-molecular-weight compound into a cell (excluding immune cells), wherein an effective amount of the nucleic acid, protein or low-molecular-weight compound is delivered into the cell (excluding immune cells) by the combined use with ultrasound exposure;
   [13-1] the preparation of [13], wherein the ultrafine bubble aqueous solution comprises one or more components selected from one or more kinds of surfactants selected from an anionic surfactant, a non-ionic surfactant, a cationic surfactant and an amphoteric surfactant, a hydrophilic resin, and a buffer;
   [13-2] the preparation of [13] or [13-1], wherein the ultrafine bubble aqueous solution consists of a non-ionic surfactant and/or a hydrophilic resin;
   [13-3] the preparation of any of [13] to [13-2], wherein the ultrafine bubble is constituted of perfluorohydrocarbon or air;
   [13-4] the preparation of any of [13] to [13-3], wherein the ultrafine bubble has an average diameter of 50 nm - 200 nm;
   [13-5] the preparation of any of [13] to [13-3], wherein the ultrafine bubble has an average diameter of 75 nm - 165 nm;
   [13-6] the preparation of any of [13] to [13-5], wherein the ultrafine bubble has a d90/d10 ratio of not more than 5;
   [13-7] the preparation of any of [13] to [13-5], wherein the ultrafine bubble has a d90/d10 ratio of not more than 4;
   [13-8] the preparation of any of [13] to [13-7], wherein the ultrafine bubble in the ultrafine bubble water or ultrafine bubble aqueous solution has a density of not less than 1.0×10⁸ bubbles/mL;
   [13-9] the preparation of any of [13] to [13-7], wherein the ultrafine bubble in the ultrafine bubble water or ultrafine bubble aqueous solution has a density of 1.0×10⁸ - 2.0×10⁹ bubbles/mL;
   [13-10] the preparation of any of [13] to [13-9], wherein ultrasound output intensity in the ultrasound generator is not more than 720 mW/cm²;
   [13-11] the preparation of any of [13] to [13-9], wherein in the ultrasound generator ultrasound output intensity is 50 - 500 mW/cm² and ultrasound frequency is 0.5 - 10 MHz;
   [13-12] the preparation of any of [13] to [13-11], wherein the target substance is a nucleic acid, a protein or a low-molecular-weight compound;
   [13-12A] the preparation of any of [13] to [13-11], wherein the target substance nucleic acid has a molecular weight of 10 mer - 30 mer;
   [13-12B] the preparation of any of [13] to [13-11], wherein the target substance protein has a molecular weight of 25 kDa - 900 kDa;
   [13-12C] the preparation of any of [1] to [9], wherein the target substance has a molecular weight of not less than 25 kDa, and the ultrafine bubble is positively charged;
   [13-12D] the preparation of any of [1] to [9], wherein the target substance has a molecular weight of less than 25 kDa; [13-13] the preparation of any of [13] to [13-12D], wherein the cell is a skeletal muscle cell or a nerve cell;
[14] a method for delivering a nucleic acid, a protein or a low-molecular-weight compound into a cell (excluding immune cells) by contacting the cell with the preparation of any of [13] to [13-13], and treating them with ultrasound;
[15] the system of any of [1] to [11], comprising a step of culturing the cell (excluding immune cells);
[16] the system of any of [1] to [11], and [15], the method of any of [12] to [12-3], or the method of [14], wherein the system or the method is performed at a temperature of -10°C - 50°C;
[17] the preparation of any of [13] to [13-13], wherein the preparation is administered to a patient at the age of 0-80;
[18] the preparation of any of [13] to [13-13], wherein the preparation is a medicament for treating a disease;
and the like.

### [Advantageous Effects of Invention]

According to the system for delivering a target substance into cells (excluding immune cells) of the present invention, the target substances such as nucleic acid, protein, low-molecular-weight compound and the like can be non-invasively and selectively introduced into cells (excluding immune cells) by irradiating ultrasound at an output intensity that does not adversely affect the living body. Therefore, a highly safe introduction system can be provided. In the system, since use of liposome is not required, addition of a special additive such as phospholipid is not necessary, production at a low cost can be realized, and the problem of antigenicity due to phospholipid does not occur.

Conventionally, it has been considered that ultrafine bubbles more severely damage cell membrane than microbubbles when crushed, and thus are not suitable for cell transfection. Surprisingly, according to the present invention, the introduction efficiency of a target substance into a cell can be remarkably increased using the ultrafine bubbles as compared with the case when microbubbles are used.

Furthermore, the ultrafine bubbles having an average diameter of not more than 200 nm are stable for a long term in ultrafine bubble water or ultrafine bubble aqueous solution.

### [Brief Description of Drawings]

Fig. 1 shows that the introduction efficiency of siRNA into skeletal muscle cells was improved by combining an ultrafine bubble aqueous solution and ultrasound. (A) shows an FAM fluorescence observation image under a fluorescence microscope. The nucleus was stained with DAPI. (B) shows the relative intensity of FAM fluorescence.
Fig. 2 shows the results of comparison of the introduction efficiency of siRNA between an ultrafine bubble aqueous solution and a conventional microbubble liposome (Sonazoid), when each was combined with ultrasound.
Fig. 3 shows that the introduction efficiency of IgG-FITC into skeletal muscle cells was improved by combining an ultrafine bubble aqueous solution and ultrasound.
   An: negatively-charged ultrafine bubble
   Cat: positively-charged ultrafine bubble
Fig. 4 shows that the results of comparison of the introduction efficiency of FITC into skeletal muscle cells by combining an ultrafine bubble aqueous solution and ultrasound with various output intensities.
The upper part of the horizontal axis of the graph shows the presence or absence and the output intensity of ultrasound, and the lower part shows the presence or absence of ultrafine bubbles.

### (Detailed Description of the Invention)

### I. The system of the present invention

The present invention provides a system for delivering a target substance into a cell (excluding immune cells), the system containing ultrafine bubble water or ultrafine bubble aqueous solution containing ultrafine bubbles with an average diameter of not more than 200 nm and not containing phospholipid, and an ultrasound generator in combination (hereinafter to be also referred to as "the system of the present invention").

In the present specification, "delivering a target substance into a cell (excluding immune cells)" means that a substance that is originally difficult to pass through a cell membrane (for example, a compound that is water-soluble and does not diffuse passively, a compound having a large molecular weight, a compound free of a selective transporter or receptor) passes through a cell membrane and is transferred into a cell. Therefore, the system of the present invention may deliver the target substance into a cell by any mechanism, and examples thereof include, but are not limited to, temporarily opening a pore in the cell membrane.

The "cell (excluding immune cells) (hereinafter to be also referred to as "non-immune cells")" to which the system of the present invention is applied is not particularly limited as long as it is a cell other than immune cells (e.g., lymphocytes such as T cell, B cell, natural killer (NK) cell, natural killer T (NKT) cell, and the like, granulocytes such as neutrophil, eosinophil, basophil and the like, monocyte, macrophage, dendritic cell, etc., and unipotent or pluripotent stem cells or progenitor cells capable of finally differentiating into them (excluding embryos and other totipotent or pluripotent stem cells). Examples thereof include nerve cell (including brain nerve cell), skeletal muscle cell, endothelial cell, epithelial cell, mesothelial cell, osteocyte, chondrocyte, adipocyte, tendon cell, eye cell, cells of major glands or organs excluding immune system (e.g., testis, liver, lung, heart, stomach, pancreas, kidney, skin, etc.), exocrine cell, endocrine cell, fibroblast, islet cell, cells derived from neuron and other neural tissue, progenitor cell and tissue stem cell having unipotency or pluripotency that can differentiate into any of the above-mentioned cells, embryo and other totipotent or pluripotent stem cells (e.g., iPS cell, ES cell, ES-like cell, embryonic germ cell, etc.), and other cells [epithelial cell, mesenchymal cell, and high purity mesenchymal stem cell (Rapidly Expanding Cells: REC)], and the like. Skeletal muscle cell, nerve cell, cardiomyocyte, hepatocyte, kidney cell, and pancreatic cell are preferred, and skeletal muscle cell or nerve cell is more preferred.

The target substance to be delivered into a non-immune cell by the system of the present invention is not particularly limited as long as it is a substance capable of imparting preferable physiological activity to the cell as a result of the delivery into the cell. Examples thereof include, but are not limited to, polymer compounds [for example, nucleic acid, small RNA/DNA such as siRNA (e.g., FAM-siRNA, manufactured by NIPPON GENE CO., LTD.), ssRNA, shRNA, miRNA, S-oligo DNA (phosphorothioate), etc.), genes (e.g., plasmid DNA, mRNA, etc.), and the like, proteins (including peptides) (e.g., antibody (e.g., IgG (e.g., Alexa-IgG, manufactured by Invitrogen)), etc.), polysaccharides (e.g., dextran, fluorescein isothiocyanate dextran, etc.) and the like], low-molecular-weight compounds (e.g., fluorescein, fluorescein sodium, etc.) and the like. Among these, polymer compounds and low-molecular-weight compounds can be preferably mentioned. They are further preferably polymer compounds. They are further preferably nucleic acid and proteins. They are particularly preferably nucleic acids and antibodies.

In the present specification, the "ultrafine bubble" may contain a gas having a general atmospheric pressure or a pressure higher than that, and the inside of the ultrafine bubble may be a vacuum. As used herein, "vacuum" means a state of a space filled with a gas having a pressure lower than the normal atmospheric pressure.

In the present invention, the "ultrafine bubble not containing phospholipid" refers to an ultrafine bubble in which the shell of the bubble does not form the structure of phospholipid bilayer.

In the present invention, the "ultrafine bubble water" refers to water containing ultrafine bubbles.

In the present invention, the "ultrafine bubble aqueous solution" refers to an aqueous solution containing ultrafine bubbles. The aqueous solution constituting the ultrafine bubble aqueous solution contains, for example, one or more kinds of substances selected from
1) one or more kinds of surfactants selected from an anionic surfactant, a non-ionic surfactant, a cationic surfactant and an amphoteric surfactant,
2) a hydrophilic resin and
3) a buffer
as components.

Examples of the "anionic surfactant" in the present invention include sodium lauryl sulfate and the like.

Examples of the "non-ionic surfactant" in the present invention include glycerol fatty acid ester (e.g., glycerol monostearate, etc.), sucrose fatty acid ester, sorbitan fatty acid ester (e.g., sorbitan monostearate, sorbitan monolaurate, etc.), polyglycerin fatty acid ester, polyoxyethylene (hydrogenated) castor oil, polyoxyethylene sorbitan fatty acid ester (e.g., sorbitan polyoxyethylene lauric acid ester (e.g., polysorbate 20, etc.), polyoxyethylene sorbitan oleic acid ester (e.g., polysorbate 80, etc.), etc.), polyethylene glycol fatty acid ester, polyoxyethylene alkyl ether (e.g., polyoxyethylene lauryl ether, etc.), polyoxyethylene polyoxypropylene alkyl ether (e.g., polyoxyethylene polyoxypropylene cetyl ether, etc.), polyoxyethylene alkylphenyl ether (e.g., polyoxyethylene nonylphenyl ether, etc.), macrogols, polyoxyethylene polyoxypropylene glycol (e.g., poloxamer 407, poloxamer 235, poloxamer 188, poloxamine, etc.) and the like. Among these, polyoxyethylene sorbitan lauric acid ester (e.g., polysorbate 20, etc.), polyoxyethylene sorbitan oleic acid ester (e.g., polysorbate 80, etc.) are preferable. Polysorbate 20 or polysorbate 80 is further preferable, and polysorbate 80 is particularly preferable.

Examples of the "cationic surfactant" in the present invention include benzalkonium chloride, benzethonium chloride, cetylpyridinium chloride, hexadecyltrimethylammonium bromide, dequalinium chloride and the like.

Examples of the "amphoteric surfactant" in the present invention include cocamidepropyl betaine, cocamidepropyl hydroxysultaine and the like.

The above-mentioned surfactants may be used alone, or two or more kinds thereof may be used in combination.

Examples of the "hydrophilic resin" in the present invention include acrylic resin (e.g., polyacrylamide, polyacryl acid, polymethyl methacrylate), vinyl resin (e.g., polyvinylpyrrolidone, poly(vinyl alcohol) (PVA), polyvinyl ethyl ether); polysaccharide (e.g., tragacanth gum, caraya gum, methylcellulose, hydroxypropylcellulose, hydroxypropylmethylcellulose, hyaluronic acid, agarose, curdlan etc.). Among these, poly(vinyl alcohol), hydroxypropylcellulose are preferable. It is more preferably poly(vinyl alcohol).

The above-mentioned hydrophilic resins alone, or two or more kinds thereof may be used in combination.

Examples of the "buffer" in the present invention include acidic buffer (e.g., acetate buffer, citrate buffer, diluted Mclivaine buffer), neutral buffer (e.g., 4-(2-hydroxyethyl)-1-piperazineethanesulfonic acid (HEPES) buffer, tris (hydroxymethyl)aminomethane (Tris) buffer, phosphate buffer, and phosphate buffered saline (PBS)). As the buffer, diluted Mclivaine buffer is preferred.

As an aqueous solution which constitutes the "ultrafine bubble aqueous solution" in the present invention is preferably an aqueous solution composed of 1) one or more kinds of surfactants selected from an anionic surfactant, a non-ionic surfactant, a cationic surfactant and an amphoteric surfactant, and/or 2) hydrophilic resin. Among these, for example, an aqueous solution composed of a non-ionic surfactant and/or a hydrophilic resin is preferable. In addition, an aqueous solution composed of a non-ionic surfactant is also preferable. An aqueous solution composed of 1) one or two kinds selected from polysorbate 80 and polysorbate 20 and/or 2) poly(vinyl alcohol) is further preferable. An aqueous solution composed of polysorbate 80 and/or poly(vinyl alcohol) is further preferable. An aqueous solution composed of polysorbate 80 is particularly preferable.

Examples of the "gas" which constitutes ultrafine bubble in the present invention include, but are not limited to, one kind or a mixture of two or more kinds selected from perfluorohydrocarbon (e.g., perfluoropropane (C₃F₈), perfluorobutane, etc.), air, nitrogen, ozone, oxygen, argon, carbon dioxide and helium and the like. Among these, perfluorohydrocarbon (e.g., perfluoropropane, perfluorobutane, etc.), air, nitrogen, ozone, oxygen, argon are preferable. Perfluorohydrocarbon (e.g., perfluoropropane, perfluorobutane, etc.), air are more preferable. When air is used, ultrafine bubbles can be produced easily at a low cost. Perfluoropropane, and air are further preferable.

The "ultrafine bubble aqueous solution" in the present invention is preferably an ultrafine bubble aqueous solution composed of (A) an aqueous solution composed of a non-ionic surfactant and/or a hydrophilic resin and (B) an ultrafine bubble constituted of one or more kinds of gas selected from perfluorohydrocarbon, air and the like. An ultrafine bubble aqueous solution composed of (A) an aqueous solution composed of 1) one or two kinds selected from polysorbate 80 and polysorbate 20 and/or 2) poly(vinyl alcohol) and (B) an ultrafine bubble constituted of one or two kinds of gas selected from perfluorohydrocarbon and air is further preferable. An ultrafine bubble aqueous solution composed of (A) an aqueous solution composed of polysorbate 80 and/or poly(vinyl alcohol) and (B) an ultrafine bubble constituted of perfluorohydrocarbon and/or air are further preferable. An ultrafine bubble aqueous solution composed of polysorbate 80 and an ultrafine bubble constituted of perfluoropropane or air is particularly preferable.

The "ultrafine bubble" in the present invention does not containing amphiphilic phospholipids such as liposome and the like. Accordingly, a safer preparation that does not show antigenicity can be provided.

The "ultrafine bubble" in the present invention has an average diameter of about not more than 200 nm. The average diameter is preferably 10 nm - 200 nm, further preferably 50 nm - 200 nm, more preferably 100 nm - 180 nm.

In another embodiment, a preferable average diameter of the average diameter of the "ultrafine bubble" in the present invention is not more than about 175 nm, further preferably 25 nm - 175 nm, more preferably 75 nm - 165 nm.

The "average diameter" in the present specification means the particle size (mode diameter) corresponding to the most frequent value of distribution (maximum value of number %).

In the present specification, the "ultrafine bubble water" or "ultrafine bubble aqueous solution" means water or aqueous solution in which gas particles (ultrafine bubbles) having a diameter of not more than 1000 nm are stably present. The ultrafine bubble water or ultrafine bubble aqueous solution in the present invention (hereinafter to be also referred to as "ultrafine bubble water, etc. in the present invention") characteristically contains ultrafine bubbles with an average diameter of not more than about 200 nm.

In the present invention, the ultrafine bubbles desirably have a uniform size. For example, when the ultrafine bubble diameters corresponding to cumulative 10% and cumulative 90% from the smaller diameter side of the ultrafine bubble number-based distribution are d10 and d90, respectively, the "d90/d10 ratio" is preferably not more than 5, further preferably not more than 4.5, more preferably not more than 4.

In the present invention, the number of ultrafine bubbles contained in the ultrafine bubble water, etc. means the number of ultrafine bubbles present in 1 mL of the ultrafine bubble water or ultrafine bubble aqueous solution, which is sometimes to be referred to as "ultrafine bubble density" in the present specification. The number of ultrafine bubbles contained in the ultrafine bubble water, etc. in the present invention is not particularly limited. The lower limit of the "ultrafine bubble density" is, for example, not less than 1.0×10⁸ bubbles/mL, preferably not less than 2.0×10⁸ bubbles/mL, more preferably not less than 2.5×10⁸ bubbles/mL. The upper limit of the "ultrafine bubble density" is, for example, not more than 2.0×10⁹ bubbles/mL, preferably not more than 1.0×10⁹ bubbles/mL. The "ultrafine bubble density" in the present invention is, for example, 1.0×10⁸-2.0×10⁹ bubbles/mL, preferably 2.0×10⁸ - 1.0×10⁹ bubbles/mL, further preferably 2.5×10⁸ - 1.0×10⁹ bubbles/mL.

The ultrafine bubble diameter (including ultrafine bubble average diameter, hereinafter the same), ultrafine bubble number-based distribution (including d90/d10 ratio, hereinafter the same), and ultrafine bubble number can be measured by a method using scattering of laser beam based on Brownian motion (e.g., NanoSight Ltd, LM20, LM10, etc.), a method based on electric resistance change (e.g., Beckman Coulter, Multisizer4, etc.), a method based on laser diffraction scattering method (e.g., Shimadzu Corporation, SALD-7100H, etc.), a method using Mie scattering (e.g., NIPPON DENSHOKU INDUSTRIES CO., LTD., NP-500T, etc.) and the like. The ultrafine bubble diameter and ultrafine bubble number-based distribution used in the present invention are those measured by a tracking method (tracking method) using laser beam scattering using NanoSight (instrument name LM10) manufactured by NanoSight Ltd., or in accordance therewith.

The ultrafine bubble diameter, ultrafine bubble number-based distribution and ultrafine bubble number may be generally measured immediately after production of ultrafine bubble water, etc., or measured after long-term storage. The ultrafine bubble diameter, ultrafine bubble number-based distribution and ultrafine bubble number of the ultrafine bubble water, etc. in the present invention are stably maintained for an extremely long time (e.g., about 6 months to 2 years), and thus, the ultrafine bubble diameter, ultrafine bubble number-based distribution and ultrafine bubble number may be measured immediately before use after sealed storage for a certain period after production of the ultrafine bubble water.

In the present specification, the "water" containing ultrafine bubbles (and "water" used as an "aqueous solution" containing ultrafine bubbles) is not particularly limited and, for example, tap water, deionized water, distilled water, sterile distilled water, purified water for injection, ultrapure water and the like can be used. For use for injection, sterile distilled water, purified water for injection and the like are preferable.

Examples of the "aqueous solution" containing ultrafine bubbles in the present specification include water containing one or more of the aforementioned components selected from one or more kinds of surfactants selected from an anionic surfactant, a non-ionic surfactant, a cationic surfactant and an amphoteric surfactant, hydrophilic resin, and buffering agent, and further containing, for example, any additive generally used in the field of pharmaceutical preparation. Examples of the "additive" include electrolyte, excipient, lubricant, binder, disintegrant, solubilizing agent, suspending agent, dispersing agent, isotonicity agent, soothing agent, antiseptic, antioxidant, colorant, sweetening agent, pH adjuster, stabilizer, acidulant, flavor, fluidizer and the like. Pharmacologically acceptable additives are preferable. One or more kinds of additives selected from suspending agent, stabilizer, dispersing agent, isotonicity agent and the like are more preferably used.

Two or more kinds of the above-mentioned additives may be used in a mixture at appropriate ratios.

These additives (including one or more kinds of surfactants selected from an anionic surfactant, a non-ionic surfactant, a cationic surfactant and an amphoteric surfactant, hydrophilic resin, and buffering agent) can also be directly dissolved in water to prepare a ultrafine bubble aqueous solution as long as they do not affect the generation, stability and the like of the ultrafine bubbles, or the ultrafine bubbles are generated in water free of additive to give ultrafine bubble water and additives are dissolved when in use to give an ultrafine bubble aqueous solution.

As the aqueous solution, any of an uncharged ultrafine bubble aqueous solution, a positively-charged ultrafine bubble aqueous solution and a negatively-charged ultrafinebubble aqueous solution can be used. They can be properly selected according to the kind of the target cell, their surrounding microenvironment, or the size or kind of the target substance, or the like. In the method of the present invention for delivery of the target substance into a non-immune cell by using the ultrafine bubble aqueous solution and ultrasound, when the target substance is a polymer compound, the ultrafine bubble aqueous solution is preferably a positively-charged ultrafine bubble aqueous solution. When the drug is a polymer compound, for example, the pH of the ultrafine bubble aqueous solution is preferably 1 - 4.

In particular, when the target substance is a protein with a high molecular weight, etc. (e.g., molecular weight not less than 25 kDa), a positively-charged ultrafine bubble aqueous solution is preferably used.

In the method of the present invention for delivery of the target substance into a non-immune cell by using the ultrafine bubble aqueous solution and ultrasound, when the molecular weight of the target substance is not high (e.g., molecular weight less than 25 kDa), both a positively-charged ultrafine bubble aqueous solution and a negatively-charged ultrafine bubble aqueous solution can be used as the ultrafine bubble aqueous solution.

The charge of the ultrafine bubble aqueous solution can be appropriately adjusted by, for example, the pH of the buffer to be used. For example, to make an ultrafine bubble aqueous solution positively-charged, the pH of the ultrafine bubble aqueous solution is preferably 1 - 4. On the other hand, to make an ultrafine bubble aqueous solution negatively-charged, the pH of the ultrafine bubble aqueous solution is preferably 7 - 14.

The production methods of ultrafine bubble water are roughly divided into a method including simultaneously generating micro-bubbles (gas particles having a diameter of about 1 - 60 µm) and ultrafine bubbles in water, and floatseparating the micro-bubbles to leave only the ultrafine bubbles, and a method including directly generating ultrafine bubbles, and the former is the mainstream at present. The former method includes a high-speed swirling flow type in which a gas is crushed by high-speed swirling to generate a large number of micro-bubbles, and the micro-bubbles are float-separated to leave ultrafine bubbles in water, a pressurized dissolution type in which a gas is pressurized to be dissolved at supersaturation, the solution is rapidly decompressed to generate micro-bubbles and ultrafine bubbles, the micro-bubbles are float-separated to leave ultrafine bubbles in water, and the like. The production methods of ultrafine bubble aqueous solution are as the same as mentioned above.

The pressurized dissolution type is preferably used as the method for producing ultrafine bubble water or ultrafine bubble aqueous solution in the present invention. For example, the following steps 1) to 3) can be mentioned; 1) in a pressurized container pressurized to about 0.2 to 0.5 MPa by a pressurization pump, a gas is forcibly dissolved in a liquid in which (i) one or more kinds of surfactants selected from an anionic surfactant, a non-ionic surfactant, a cationic surfactant and an amphoteric surfactant, (ii) a hydrophilic resin and/or (iii) buffer solution; 2) flash operation in water through a nozzle is performed to release the depressurized and supersaturated gas into the waste water as micro-bubbles or ultrafine bubbles, whereby a mixture of micro-bubble water and ultrafine bubble water is produced; 3) aeration is discontinued and the mixture is left standing to allow micro-bubbles to be naturally separated by floating. As a result, clear ultrafine bubble water with only ultrafine bubbles remaining therein is produced.

Examples of the ultrafine bubble generator used in producing ultrafine bubble water or ultrafine bubble aqueous solution in the present invention include pressurized dissolution type apparatus (e.g., nanoGALF^{™} manufactured by IDEC, OM4-MD5-045 manufactured by AURA TEC CO., LTD., microbubble generator manufactured by Nikuni Corporation, etc.), high-speed swirling flow type apparatus (e.g., YJ manufactured by Bi-clean, micro-bubble generator manufactured by AQUA AIR, MICROBLADE manufactured by ROYAL ELECTRIC CO., LTD., etc.) and the like. As the ultrafine bubble generator, a pressurized dissolution type apparatus (e.g., nanoGALF^{™} manufactured by IDEC) is preferable.

In the present invention, one or more kinds of surfactants selected from an anionic surfactant, a non-ionic surfactant, a cationic surfactant and an amphoteric surfactant, hydrophilic resin and/or buffer solution are used for the production of ultrafine bubble water or ultrafine bubble aqueous solution, whereby the number of ultrafine bubbles in the above-mentioned ultrafine bubble water or ultrafine bubble aqueous solution can be increased.

The content of one or more kinds of surfactants selected from an anionic surfactant, a non-ionic surfactant, a cationic surfactant and an amphoteric surfactant, hydrophilic resin and/or buffer solution used in the present invention in water is not particularly limited. The upper limit is preferably not more than 50% (W/V), more preferably not more than 20% (W/V), further preferably not more than 10% (W/V). The lower limit is preferably not less than 0.01% (W/V), more preferably not less than 0.05% (W/V), further preferably not less than 0.1%(W/V). As used herein, (W/V) means g/mL.

When two or more kinds of the surfactant, hydrophilic resin and buffer solution are used in combination, the total amount thereof is the content in water.

The ultrafine bubble water, etc. in the present invention which is produced as mentioned above is tightly sealed in a vial or ampoule and preserved. The preservation is preferably performed under shading conditions. The preservation temperature is preferably not more than room temperature, and not more than 10°C is more preferable.

The ultrafine bubble water or ultrafine bubble aqueous solution in the present invention is preferably produced in the presence of one or more kinds of surfactants selected from an anionic surfactant, a non-ionic surfactant, a cationic surfactant and an amphoteric surfactant, hydrophilic resin and/or buffer solution. Therefore, the number of ultrafine bubbles in ultrafine bubble water, etc. can be maintained at not less than 1.0×10⁸ bubbles/mL for a period when maintenance of the action effect of the ultrafine bubble water, etc. in the present invention (e.g., cell membraneperforating effect on non-immune cell when ultrafine bubble water, etc. and ultrasonic treatment are used in combination, effect of increasing the delivery of an the target substance into a non-immune cell by using ultrafine bubble water, etc. and ultrasound, etc.) is required (e.g., when used as a base for an the target substance that is desired to be delivered through cell membrane, etc., the effective period thereof (e.g., not less than 3 months, more preferably not less than 6 months, further preferably not less than one year)).

The ultrafine bubble water, etc. in the present invention can be sterilized by heating, and the number of ultrafine bubbles can be maintained at not less than 1.0×10⁸ bubbles/mL even after heat sterilization.

When the number of ultrafine bubbles in the ultrafine bubble water, etc. in the present invention is not less than 1.0×10⁸ bubbles/mL, a superior antibacterial action and a preservation effect resulting therefrom are exhibited. Therefore, they are also useful as a base for a liquid pharmaceutical preparation of a multiple administration type which is repeatedly administered from the same container for a certain period of time, for example, injection (e.g., subcutaneous injection, intravenous injection, intramuscular injection, intraperitoneal injection, drip transfusion, intracerebral injection, intra-cerebrospinal fluid injection, intraocular injection, etc.).

The ultrafine bubble water, etc. in the present invention is preferably produced in the presence of one or more kinds of surfactants selected from an anionic surfactant, a non-ionic surfactant, a cationic surfactant and an amphoteric surfactant (preferably polysorbate 80 and/or polysorbate 20, further preferably polysorbate 80), and the ultrafine bubbles in the produced ultrafine bubble water, etc. have a smaller average diameter and more uniform size (i.e., d90/d10 ratio is small). Therefore, a safer effect of delivering the target substance into a non-immune cell is obtained in the system of the present invention.

As an "ultrasound generator" used in the system of the present invention, any can be used as long as it can generate ultrasonic wave meeting conditions sufficient to deliver a target substance into a non-immune cell when used in combination with the ultrafine bubble water, etc. in the present invention. For example, any apparatus conventionally used for ultrasound diagnosis in the clinical situation, a commercially available ultrasound gene transfer apparatus (e.g., Sonitron GTS (manufactured by Nepa Gene Co., Ltd.), etc.) and the like can be appropriately used.

The conditions of the "ultrasound generator" in the present invention include, for example, an ultrasound output intensity (acoustic energy passing through a unit area (cm²) perpendicular to the traveling direction of sound waves in a unit time) of not less than 10 mW, preferably not less than 30 mW, more preferably not less than 50 mW (for example, not less than 100 mW, not less than 150 mW, not less than 200 mW, etc.). The upper limit of the output intensity is not particularly set. Considering that the system of the present invention takes one of the aims to deliver a target substance into a target cell in vivo in mammals including human, a range that does not adversely affect the animals (e.g., cytotoxicity) is preferable as the upper limit. For example, the requirement of "not exceeding 720 mW/cm²" (same as the upper limit of Track 3 of US FDA) was added to the third party certification criteria (2005) of the revised Pharmaceutical Affairs Law, and ultrasound diagnosis apparatuses in Japan are controlled so as not to exceed the upper limit. Thus, the upper limit of the ultrasound output intensity in the system of the present invention is preferably 720 mW/cm². Preferably, the output intensity is not more than 500 mW/cm² (for example, not more than 450 mW/cm², not more than 400 mW/cm², not more than 300 mW/cm² or the like). Accordingly, the ultrasound output intensity is preferably 50 - 720 mW/cm², more preferably 50 - 500 mW/cm², further preferably 55 - 450 mW/cm². The ultrasound output intensity used for conventional gene transfer is significantly higher than the above-mentioned criteria for ultrasound diagnosis applications (e.g., 1.5 - 2.5W/cm²), thus posing a high safety risk. In the system of the present invention, a target substance can be efficiently delivered into a non-immune cell with a small output intensity (preferably 50 - 500 mW/cm²) regardless of the molecular weight of the target substance, and it is an extremely safe deliver system.

As the conditions of the "ultrasound generator" in the present invention, the ultrasound frequency is not particularly limited and can be appropriately selected, for example, within the range of 0.5 - 10 MHz. The frequency that is widely adopted at present is about 1 MHz. However, since higher frequencies are considered to have less adverse influence on the body, the frequency can be appropriately selected within the range of 1 to 5 MHz, more preferably 1 to 3 MHz, further preferably 1 to 2.5 MHz.

As the conditions for using the "ultrasound generator" in the present invention, the ultrasound exposure time is not particularly limited as long as it is sufficient to deliver a target substance into a non-immune cell, and varies depending on the ultrasound output intensity. For example, even when the output intensity is 50 mW/cm², the delivery of a target substance into a non-immune cell can be achieved by an exposure time of 10 seconds. The ultrasound exposure time may be, for example, 1 to 60 seconds, preferably 1 to 30 seconds, more preferably 1 to 20 seconds, further preferably 1 to 10 seconds.

As the conditions for using the "ultrasound generator" in the present invention, for example, a combination of (i) ultrasound output intensity of 50 to 720 mW/cm² (preferably 50 to 500 mW/cm²), (ii) ultrasound frequency of 0.5 to 10 MHz, and (iii) ultrasound exposure time of 1 to 60 seconds can be mentioned. Among these, a combination of (i) ultrasound output intensity of 50 to 500 mW/cm², (ii) ultrasound frequency of 1 to 5 MHz, and (iii) ultrasound exposure time of 1 to 60 seconds, and the like are preferable, and a combination of (i) ultrasound output intensity of 55 to 450 mW/cm², (ii) ultrasound frequency of 1 to 2.5 MHz, and (iii) ultrasound exposure time of 1 to 10 seconds, and the like are more preferable.

When the system of the present invention is used for the transfection of non-immune cells isolated from an animal, it may include a step of culturing the non-immune cells after a transfection treatment. Therefore, the system of the present invention may further include a means for culturing non-immune cells (e.g., culture container (e.g., dish, flask, etc.), medium, culture apparatus (e.g., CO₂ incubator, etc.)). The above-mentioned culture step also includes a passage culture step. When cells are passage cultured by the system of the present invention, the expression intensity of the target substance introduced into the cell can be maintained.

### II. The delivery-increasing method of the present invention

The present invention also provides a method for increasing the delivery of a nucleic acid, a protein or a low-molecular-weight compound into a non-immune cell, by using ultrafine bubble water or ultrafine bubble aqueous solution containing ultrafine bubbles with an average diameter of not more than 200 nm and not containing phospholipid, and ultrasound (hereinafter to be also referred to as "the delivery-increasing method of the present invention"). The method includes administering ultrafine bubble water, etc. in the present invention to a subject to deliver same to the vicinity of the non-immune cell, and performing ultrasound exposure to the non-immune cell, whereby the nucleic acid, protein or the low-molecular-weight compound is delivered into the non-immune cell. As used herein, the "non-immune cell" means the same as that defined for the above-mentioned system of the present invention.

As the ultrafine bubble water or ultrafine bubble aqueous solution to be used in "the delivery-increasing method of the present invention", the above-mentioned ultrafine bubble water, etc. in the present invention can be used. The ultrasound exposure in "the delivery-increasing method of the present invention" can be performed under conditions similar to those for the use of the ultrasound generator in the above-mentioned system of the present invention.

The nucleic acid, protein or low-molecular-weight compound whose delivery into a non-immune cell is increased by the delivery-increasing method of the present invention is not particularly limited, and may be, for example, a substance capable of imparting preferable physiological activity to a non-immune cell as a result of the delivery into the cell. Examples of the nucleic acid include, but are not limited to, small RNA/DNA such as siRNA (e.g., FAM-siRNA, manufactured by NIPPON GENE CO., LTD.), ssRNA, shRNA, miRNA, S-oligo DNA (phosphorothioate), etc.), genes (e.g., plasmid DNA, mRNA, etc.), and the like. Examples of the protein include antibody (e.g., IgG (e.g., Alexa-IgG, manufactured by Invitrogen Corp.), etc.), peptide and the like. Examples of the low-molecular-weight compound include, but are not limited to, known or novel medicament compounds, fluorescent substances such as fluorescein, fluorescein sodium and the like. Among these, small RNA/DNA and the like such as siRNA and antisense oligo nucleic acid, are preferred as the nucleic acid, antibody and the like are preferred as the protein, and medicament compounds, fluorescence dye, and the like are preferred as the low-molecular-weight compound.

The number of nucleotides in the nucleic acid and the molecular weight of the protein are not particularly limited. For example, in the case of nucleic acid (small RNA/DNA such as siRNA, antisense oligo nucleic acid, and the like), it is 10 mer - 30 mer, preferably 15 mer - 25 mer; in the case of protein, it is 25 kDa - 900 kDa, preferably 25 kDa - 320 kDa.

### III. The preparation of the present invention

The present invention also provides a preparation comprising a combination of a nucleic acid, a protein or a low-molecular-weight compound, and ultrafine bubble water or ultrafine bubble aqueous solution comprising ultrafine bubble with an average diameter of not more than 200 nm and not containing phospholipid, for delivering the nucleic acid, protein or low-molecular-weight compound into a non-immune cell, wherein an effective amount (amount at which non-immune cell exerts a desired effect) of the nucleic acid, protein or low-molecular-weight compound is delivered into the non-immune cell by the combined use with ultrasound exposure (hereinafter to be also referred to as "the preparation of the present invention"). As used herein, the "non-immune cell" means the same as that defined for the above-mentioned system of the present invention.

In the preparation of the present invention, the nucleic acid, protein or low-molecular-weight compound can be the nucleic acid, protein or low-molecular-weight compound exemplified in the above-mentioned "delivery-increasing method of the present invention". As the ultrafine bubble water or ultrafine bubble aqueous solution in the preparation of the present invention, the ultrafine bubble water or ultrafine bubble aqueous solution described in the above-mentioned "system of the present invention" may be used.

In the formulation of the present invention, a nucleic acid, protein or low-molecular-weight compound may be mixed with the ultrafine bubble water, etc. in the present invention and administered to the subject as a single preparation, or they may be separately formulated and administered at the same time or at different times by the same or different routes as long as they can simultaneously coexist adjacent to a non-immune cell.

When the nucleic acid, protein or low-molecular-weight compound and the ultrafine bubble water, etc. in the present invention are separately formulated, the nucleic acid, protein or low-molecular-weight compound can be mixed with a pharmaceutically acceptable carrier in an amount that can be tolerated by human or other mammals.

Examples of the pharmaceutically acceptable carrier include pH adjusters such as monosodium phosphate, dipotassium phosphate, disodium phosphate, monopotassium phosphate, sodium hydroxide, hydrochloric acid and the like; antibiotics such as kanamycin sulfate, erythromycin lactobionate, penicillin G potassium and the like; stabilizers such as lactose, potassium glutamate, D-sorbitol, aminoacetic acid, human serum albumin and the like; colorants such as phenol red and the like; isotonicity agents such as sodium chloride, potassium chloride and the like, and the like.

The content of the nucleic acid, protein or low-molecular-weight compound in the preparation of the present invention is not particularly limited as long as preferable properties such as desired physiological activity and the like can be imparted to the non-immune cells when they are delivered into the cells by ultrasound exposure. For example, the content may be 0.0001 mg - 1000 mg, preferably 0.001 - 10 mg. On the other hand, the amount of ultrafine bubble water, etc. is not particularly limited as long as it is sufficient to deliver a nucleic acid, a protein or a low-molecular-weight compound into a non-immune cell by ultrasound exposure. For example, an amount permitting the delivery of ultrafine bubbles such that the ultrafine bubble density near the non-immune cell is 1×10⁸ - 10×10⁸ bubbles/mL, preferably 2×10⁸-5×10⁸ bubbles/mL.

### IV. The delivery method of the present invention

The present invention also provides a method for delivering a nucleic acid or a protein into a non-immune cell, including contacting the cell with the above-mentioned preparation of the present invention, and treating the cell with ultrasound. As used herein, the "non-immune cell" means the same as that defined for the above-mentioned system of the present invention.

The target to which "the delivery-increasing method of the present invention" or "the delivery method of the present invention" is applied is not particularly limited as long as it is a non-immune cell collected from the living body (e.g., nerve cell (including brain nerve cell), skeletal muscle cell, endothelial cell, epithelial cell, mesothelial cell, osteocyte, chondrocyte, adipocyte, tendon cell, eye cell, cells of major glands or organs excluding immune system(e.g., testis, liver, lung, heart, stomach, pancreas, kidney, skin, etc.), exocrine cell, endocrine cell, fibroblast, islet cell, cells derived from neuron and other neural tissue, progenitor cell and tissue stem cell having unipotency or pluripotency that can differentiate into any of the above-mentioned cells, embryo and other totipotent or pluripotent stem cells (e.g., iPS cell, ES cell, ES-like cell, embryonic germ cell, etc.), and other cells [epithelial cell, mesenchymal cell, and high purity mesenchymal stem cell (Rapidly Expanding Cells: REC)], and the like) (to be also referred to as "ex vivo non-immune cell" in the present specification) or a non-immune cell in the tissue or the body of an animal containing same (to be also referred to as "in vivo non-immune cell" in the present specification). For example, human and other mammals (e.g., mouse, rat, rabbit, dog, cat, bovine, horse, swine, monkey, etc.), a non-immune cell derived from them or a tissue containing same, and the like can be mentioned. The means for administering the ultrafine bubble water or the like in the present invention to the subject is not particularly limited as long as it is an administration route capable of delivering the ultrafine bubbles to the vicinity of non-immune cells. The ex vivo non-immune cells and in vivo non-immune cells are described separately in the following.

### (a) Delivery of target substance to ex vivo non-immune cell

According to the delivery method of the present invention, a nucleic acid, a protein or a low-molecular-weight compound is delivered into ex vivo non-immune cells by combining ultrafine bubble water, etc. and ultrasound in the present invention. As a result, ex vivo non-immune cells imparted with favorable properties such as physiological activity and the like by the action of the nucleic acid, protein or low-molecular-weight compound can be produced.

Ex vivo non-immune cells can be collected from tissues/organs of human or non-human mammals that contain the cells. When ex vivo non-immune cells (e.g., ex vivo skeletal muscle cell, ex vivo nerve cell) into which the preparation of the present invention has been introduced are used for the treatment of diseases such as cancer and the like, the cell population is preferably collected from a subject to be treated itself or a donor matched to the MHC type of the subject to be treated.

Ex vivo non-immune cells may be undifferentiated cells such as pluripotent stem cells, tissue stem cells other than the immune system, and the like. For example, embryonic stem cell (ES cell), induced pluripotent stem cell (iPS cell), embryonal carcinoma cell (EC cell), embryonic germ cell (EG cell), neural stem cell, skeletal muscle progenitor cell, and the like can be mentioned. Undifferentiated cells such as pluripotent stem cell and the like can be differentiated into various non-immune cells, for example, skeletal muscle cell, nerve cell, and the like by a method known per se.

The method for contacting the preparation of the present invention with ex vivo non-immune cells is not particularly limited and, for example, the preparation of the present invention may be added to a general medium for non-immune cells. Ultrasound exposure can be performed on ex vivo non-immune cells by using the ultrasound generator and irradiation conditions described with regard to the system of the present invention.

### (b) Delivery of target substance to in vivo non-immune cell

According to the delivery method of the present invention, the preparation of the present invention is administered to mammals (human or other mammal (e.g., mouse, rat, hamster, rabbit, cat, dog, bovine, sheep, monkey), preferably human) and tissue or organ (e.g., skeletal muscle, brain, etc.) containing non-immune cells is exposed to ultrasound, whereby a nucleic acid, a protein or a low-molecular-weight compound is introduced into the non-immune cell (e.g., skeletal muscle cell, nerve cell, etc.) in the animal body and the action effect of the target substance is achieved. For example, an antisense nucleic acid capable of skipping a mutation that causes MDS is delivered into in vivo skeletal muscle cells of a muscular dystrophy (MDS) patient and expressed in the cells, whereby cytotoxicity is reduced and a treatment effect on the disease can be exhibited.

The preparation of the present invention in the form of, for example, injection can deliver a nucleic acid, protein or low-molecular-weight compound, ultrafine bubble water, and the like to the vicinity of non-immune cells in vivo by subcutaneous injection, intravenous injection, intramuscular injection, intraperitoneal injection, drip injection, intracerebral injection, intra-cerebrospinal fluid injection or the like. In addition, the ultrasound exposure can be performed by an operation conventionally used for ultrasound diagnosis, by substituting the target region with a tissue or organ containing non-immune cell in vivo.

In another embodiment, a mixed solution of ultrafine bubbles having different average diameters is administered, and plural ultrasonic waves of different frequencies or output intensities are combined to allow a nucleic acid, protein or low-molecular-weight compound to pass through the cell membrane barrier in multiple stages and be successively delivered into a target non-immune cell.

When "the delivery-increasing method of the present invention" or "the delivery method of the present invention" is applied to an ex vivo non-immune cell, that is, a non-immune cell isolated from an animal, or a non-immune cell obtained by inducing differentiation of a stem cell such as iPS cell and the like, using a method known per se, a step of culturing the non-immune cell after a transfection treatment may be included. The culturing step can be performed using a culture container (e.g., dish, flask, etc.) and a culture apparatus (e.g., CO₂ incubator, etc.) known per se, in a medium generally used for maintenance culture of non-immune cells and inducing differentiation of undifferentiated stem cell into other non-immune cell. As long as the transfection in the present invention is performed on non-immune cells (also including pluripotent stem cells and tissue stem/progenitor cell other than immune system), the present invention does not eliminate induction of differentiation from non-immune cells or induction of immune cell by direct reprogramming after transfection.

In addition, the above-mentioned culture step also includes a passage culture step. When cells are passage cultured by "the delivery-increasing method of the present invention" or "the delivery method of the present invention", the expression intensity of the target substance introduced into the cell can be maintained.

### V. Medicament containing ex vivo non-immune cell into which

target substance has been introduced by the present invention The ex vivo non-immune cell into which the target substance has been introduced using the system of the present invention or by the delivery method of the present invention provides desired effects (e.g., acquisition of new physiological activity) by the action of the target substance. Therefore, it can be directly formulated as a pharmaceutical composition, or by mixing with known pharmaceutically acceptable carriers (including excipient, diluent, filler, binder, lubricant, flow aid, disintegrant, surfactant, etc., and the like), conventionally-used additives, and the like. Excipients are well known to those of skill in the art, and, adjuvants such as wetting agent or emulsifier and the like, and pH buffering agents can also be used. Furthermore, formulation adjuvants such as suspending agent, preservative, stabilizer, dispersing agent, and the like, and the like may also be used. The above-mentioned pharmaceutical composition may be in a dry form to be reconstituted with a suitable sterile liquid before use. The pharmaceutical composition can be systemically or locally administered orally or parenterally depending on the form of preparation (oral agents such as tablet, pill, capsule, powder, granule, syrup, emulsion, suspension and the like; parenteral agents such as injection, drip transfusion, external preparation, suppository and the like) and the like. In the case of parenteral administration, intravenous administration, intradermal administration, subcutaneous administration, rectal administration, transdermal administration and the like are possible. In addition, when used in the injection form, an acceptable buffering agent, solubilizing agent, isotonic agent, and the like can also be added.

A medicament containing an ex vivo non-immune cell into which the target substance has been introduced by the present invention can be a prophylactic or therapeutic agent for various diseases, and examples of the target disease include cancer and hereditary diseases. For example, when the target substance is an antisense nucleic acid capable of skipping a mutation that causes MDS, the expression of highly cytotoxic splicing variants is suppressed and the cytotoxicity can be reduced in the ex vivo skeletal muscle cell into which the target substance has been introduced and expressed therein.

A medicament containing an ex vivo non-immune cell into which the target substance has been introduced by the present invention can be safely administered to human or other mammals (e.g., mouse, rat, hamster, rabbit, cat, dog, bovine, sheep, monkey), preferably human.

The medicament containing an ex vivo non-immune cell into which the target substance has been introduced by the present invention is preferably administered parenterally to a subject. The parenteral administration method includes intravenous, intraarterial, intramuscular, intraperitoneal, and subcutaneous administration methods, and the like. The dose is appropriately selected according to the condition, body weight, age, etc. of the subject. Generally, it is administered such that the cell number is generally 1×10⁶-1×10¹⁰ cells, preferably 1×10⁷ - 1×10⁹ cells, more preferably 5×10⁷ - 5×10⁸ cells, per administration to a subject with a body weight of 60 kg. In addition, it may be administered once or administered in multiple portions.

The present invention is further described in the following by Reference Examples and Examples; however, the present invention is not limited to them in any sense.

In the following Reference Examples and Examples, "%" indicates weight/volume % unless otherwise specified.

### [Example]

### Reference Example 1: Preparation of ultrafine bubble water

Polysorbate 80 (2 g) (0.1% polysorbate 80) was dissolved in water for injection (2L) or diluted Mclivaine buffer (pH3.0, 2 L), and ultrafine bubble aqueous solution was prepared using an ultrafine bubble generator (nanoGALF^{™} FZ1N-02) manufactured by IDEC with the following settings. Positively-charged ultrafine bubble can be produced when an acidic buffer (diluted Mclivaine buffer: pH 3.0) is used, and negatively-charged ultrafine bubble can be produced when water for injection is used.
gas used for preparation: air (Example 3), C₃F₈ (Examples 1,2,4)
bubble water flow: about 4.0 L/min
dissolution pressure: 300 KPa ± 5%

The prepared ultrafine bubble water was subjected to high-pressure vapor sterilization using an autoclave as appropriate at 121 - 124°C for 30 min. After sterilization, ultrafine bubble average diameter, ultrafine bubble density and d90/d10 ratio were measured by a tracking method utilizing laser beam scattering using LM10, NanoSight Ltd.

The results are shown below.
ultrafine bubble average diameter: 120 nm ± 16 nm
ultrafine bubble density: 4×10⁸ bubbles/mL
d90/d10 ratio: 3.3

### Example 1 Introduction of siRNA into skeletal muscle cell

C₂C₁₂ cells (3×10⁴ cells, 0.5 mL) in 10% FBS-containing DMEM medium were seeded in 48 wells. After removing the medium, 0.5 mL of ultrafine bubble/DMEM medium containing 5 µg of siRNA fluorescently labeled with FAM (FAM-siRNA) was added. An ultrafine bubble/DMEM medium prepared by adding 1 L of the ultrafine bubble aqueous solution prepared in Reference Example 1 to a DMEM medium powder, and adding 2 g/L NaHCO₃ and 10% FBS was used as a transfection medium.

After adding the FAM-siRNA-containing medium, ultrasound exposure was performed for 10 sec at a frequency of 1 MHz and an output intensity of 500 mW/cm² using an ultrasound generator (NEPAGENE). After ultrasound exposure, the cells were cultured for 2 hr, then the transfection medium was removed, and the cells were cultured in DMEM medium (culture medium) for 48 hr.

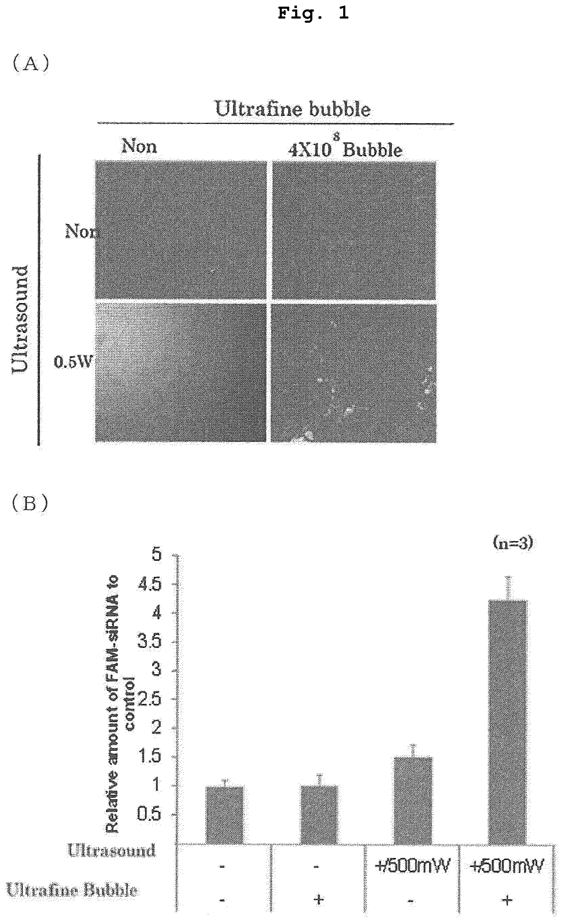

After completion of the culture, the cells were stained with DAPI and observed under a fluorescence microscope (manufactured by KEYENCE). The results are shown in Fig. 1A. Delivery of siRNA into the cell was not observed with ultrafine bubbles alone. Ultrasound exposure showed a slight intracellular delivery of siRNA. The introduction efficiency of siRNA into the cell was remarkably increased by combining ultrafine bubbles and ultrasound.

After completion of the culture, the cells were collected, completely lysed with 0.5 mL of cell lysis solution, and the fluorescent intensity of FAM-siRNA was measured with a spectrofluoro-photometer. The amount of FAM-siRNA uptake was evaluated as a relative amount by proportional calculation with the measured value of Control (ultrafine bubbles alone) as 1. The results are shown in Fig. 1B. The results of fluorescence microscope observation were confirmed quantitatively.

Fig. 1 shows the results of a negatively-charged ultrafine bubble (produced using water for injection). In the case of siRNA, similar effects are obtained with a positively-charged ultrafine bubble (produced using diluted Mclivine buffer (pH 3.0)).

### Example 2 Comparative introduction with microbubble

C₂C₁₂ cells (3×10⁴ cells, 0.5 mL) in 10% FBS-containing DMEM medium were seeded in 48 wells. After removing the medium, 0.5 mL of ultrafine bubble/DMEM medium or microbubble/DMEM medium containing 5 µg of siRNA fluorescently labeled with FAM (FAM-siRNA) was added. An ultrafine bubble/DMEM medium and microbubble/DMEM medium prepared by adding 1 L of the ultrafine bubble aqueous solution prepared in Reference Example 1 or commercially available microbubble water to a DMEM medium powder, and adding 2 g/L NaHCO₃ and 10% FBS was used as a transfection medium. As the microbubble water, Sonazoid (average diameter: 3 µm) containing phosphatidyl serine, which is a phospholipid, in the constituent component was used.

After adding each FAM-siRNA-containing medium, ultrasound exposure was performed for 10 sec at a frequency of 1 MHz and an output intensity of 500 mW/cm² using an ultrasound generator (NEPAGENE). After ultrasound exposure, the cells were cultured for 2 hr, then the transfection medium was removed, and the cells were cultured in DMEM medium (culture medium) for 48 hr.

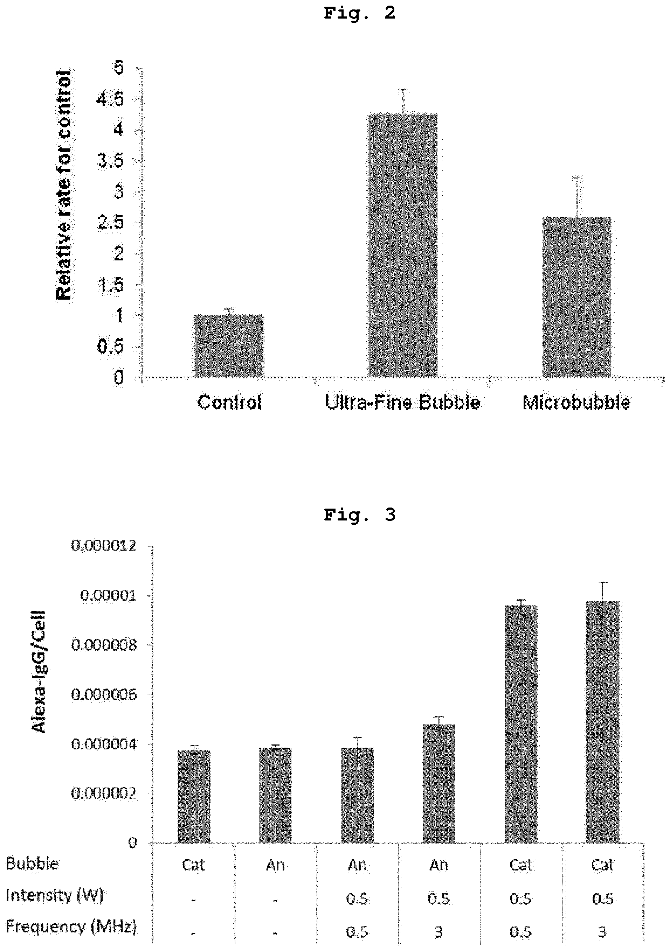

After completion of the culture, the cells were collected, completely lysed with 0.5 mL of cell lysis solution, and the fluorescent intensity of FAM-siRNA was measured with a spectrofluoro-photometer. The amount of FAM-siRNA uptake was evaluated as a relative amount by proportional calculation with the measured value of Control (ultrafine bubbles alone) as 1. The results are shown in Fig. 2. The ultrafine bubble water more remarkably increased the introduction efficiency of siRNA into the cell than the microbubble water.

Fig. 2 shows the results of a negatively-charged ultrafine bubble (produced using water for injection). In the case of siRNA, similar effects are obtained with a positively-charged ultrafine bubble (produced using diluted Mclivine buffer (pH3.0)).

### Example 3 Introduction of protein into skeletal muscle cell

C₂C₁₂ cells (6×10⁴ cells, 1 mL) in 10% FBS-containing DMEM medium were seeded in 48 wells. After removing the medium, 1 mL of a solution of IgG-FITC diluted 100-fold with ultrafine bubble/DMEM medium was added. An ultrafine bubble/DMEM medium prepared by adding 1 L of the ultrafine bubble aqueous solution prepared in Reference Example 1 to a DMEM medium powder, and adding 2 g/L NaHCO₃ and 10% FBS was used as a transfection medium.

After adding the IgG-FITC-containing medium, ultrasound exposure was performed for 10 sec at an output intensity of 500 mW/cm² and a frequency of 0.5 MHz or 3 MHz and using an ultrasound generator (NEPAGENE). After ultrasound exposure, the cells were cultured for 2 hr, then the transfection medium was removed, and the cells were cultured in DMEM medium (culture medium) for 48 hr.

After completion of the culture, the fluorescent intensity of IgG-FITC was measured with a spectrofluoro-photometer. The number of surviving cells was also measured, converted into the fluorescent intensity per surviving cell, and compared. The results are shown in Fig. 3. The introduction efficiency of IgG-FITC into the cell was remarkably increased by combining ultrafine bubbles and ultrasound. In the range examined, the frequency of the ultrasound did not affect the introduction efficiency.

### Example 4 Introduction of low-molecular-weight compound into skeletal muscle cell

C₂C₁₂ cells (3×10⁴ cells, 0.5 mL) in 10% FBS-containing DMEM medium were seeded in 48 wells. After removing the medium, 0.5 mL of ultrafine bubble/DMEM medium containing 0.38 µg of FITC was added. An ultrafine bubble/DMEM medium prepared by adding 1 L of the ultrafine bubble aqueous solution prepared in Reference Example 1 to a DMEM medium powder, and adding 2 g/L NaHCO₃ and 10% FBS was used as a transfection medium.

After adding the FITC-containing medium, ultrasound exposure was performed for 10 sec at a frequency of 1 MHz and output intensities of 100, 250, 500 mW/cm² and using an ultrasound generator (NEPAGENE). After ultrasound exposure, the cells were cultured for 2 hr, then the transfection medium was removed, and the cells were cultured in DMEM medium (culture medium) for 48 hr.

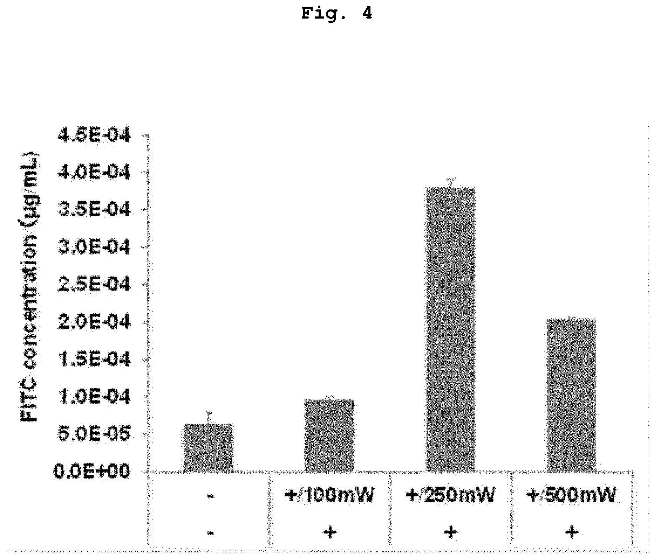

After completion of the culture, the cells were collected, completely lysed with 0.5 mL of cell lysis solution, and the fluorescent intensity of FITC was measured with a spectrofluoro-photometer. The amount of FITC uptake was evaluated as a relative amount by proportional calculation with the measured value of Control (ultrafine bubbles alone) as 1. The results are shown in Fig. 4. The introduction efficiency of FITC into the cell was remarkably increased by combining ultrafine bubbles and ultrasound. The output intensity of ultrasound greatly affected the introduction efficiency, and an increasing tendency was observed even at 100 mW/cm². The introduction efficiency of FITC increased significantly at 250 or 500 mW/cm², and particularly superior introduction efficiency was shown at 250 mW/cm².

Fig. 4 shows the results of a negatively-charged ultrafine bubble (produced using water for injection). In the case of low-molecular-weight compound, similar effects are obtained with a positively-charged ultrafine bubble (produced using diluted Mclivine buffer (pH 3.0)).

### [Industrial Applicability]

The system of the present invention can efficiently deliver a target substance such as nucleic acid, protein, low-molecular-weight compound, or the like into a non-immune cell by ultrasound exposure at a low output intensity, and thus can provide a highly safe system for drug delivery into a non-immune cell. As compared with the use of conventional microbubbles, the introduction efficiency of a target substance into a cell can be remarkably increased using the ultrafine bubbles. Furthermore, since the system does not require use of liposome, it can be manufactured at a low cost and is highly safe. From the above, the system of the present invention is extremely useful as a novel DDS into a non-immune cell.

## Claims

1. A system for delivering a target substance into a cell (excluding immune cells), comprising ultrafine bubble water or ultrafine bubble aqueous solution comprising ultrafine bubbles with an average diameter of not more than 200 nm and not containing phospholipid, and an ultrasound generator in combination.

2. The system according to claim 1, wherein the ultrafine bubble aqueous solution comprises one or more components selected from one or more kinds of surfactants selected from an anionic surfactant, a non-ionic surfactant, a cationic surfactant and an amphoteric surfactant, a hydrophilic resin, and a buffer.

3. The system according to claim 1, wherein the ultrafine bubble aqueous solution consists of a non-ionic surfactant and/or a hydrophilic resin.

4. The system according to claim 1, wherein the ultrafine bubble is constituted of perfluorohydrocarbon or air.

5. The system according to claim 1, wherein the ultrafine bubble has an average diameter of 50 nm - 200 nm.

6. The system according to claim 1, wherein the ultrafine bubble has a d90/d10 ratio of not more than 5.

7. The system according to claim 1, wherein the ultrafine bubble in the ultrafine bubble water or ultrafine bubble aqueous solution has a density of not less than 1.0×10⁸ bubbles/mL.

8. The system according to claim 1, wherein ultrasound output intensity in the ultrasound generator is not more than 720 mW/cm².

9. The system according to claim 1, wherein in the ultrasound generator, ultrasound output intensity is 50 - 500 mW/cm² and ultrasound frequency is 0.5 - 10 MHz.

10. The system according to claim 1, wherein the target substance is a nucleic acid, a protein or a low-molecular-weight compound.

11. The system according to claim 1, wherein the cell is a skeletal muscle cell or a nerve cell.

12. A method for increasing the delivery of a nucleic acid, a protein or a low-molecular-weight compound into a cell (excluding immune cells) by using ultrafine bubble water or ultrafine bubble aqueous solution comprising ultrafine bubble with an average diameter of not more than 200 nm and not containing phospholipid, and ultrasound.

13. A preparation comprising a combination of a nucleic acid, a protein or a low-molecular-weight compound, and ultrafine bubble water or ultrafine bubble aqueous solution comprising ultrafine bubble with an average diameter of not more than 200 nm and not containing phospholipid, for delivering the nucleic acid, the protein or the low-molecular-weight compound into a cell (excluding immune cells), wherein an effective amount of the nucleic acid, the protein or the low-molecular-weight compound is delivered into the cell (excluding immune cells) by the combined use with ultrasound exposure.

14. A method for delivering a nucleic acid, a protein or a low-molecular-weight compound into a cell (excluding immune cells) by contacting the cell with the preparation according to claim 13, and treating them with ultrasound.
